# EUROPÄISCHE PATENTSCHRIFT

(11) **EP 1 967 231 B1**
(45) Veröffentlichungstag und Bekanntmachung des Hinweises auf die Patenterteilung: **30.12.2009**
(21) Anmeldenummer: 08102179.2
(22) Anmeldetag: 29.02.2008
(51) Int. Cl.: A61N 5/10

(54) **Vorrichtung und Verfahren für die Qualitätsüberprüfung einer Partikeltherapieanlage**
Device and method for quality testing a particle therapy assembly
Dispositif et procédé de vérification de la qualité d'une installation de thérapie à particules

(30) Priorität: 07.03.2007 DE 102007011153
(43) Veröffentlichungstag der Anmeldung: 10.09.2008
(73) Patentinhaber: SIEMENS AKTIENGESELLSCHAFT, 80333 München (DE)
(72) Erfinder: Gunzert-Marx, Konstanze, 91054, Erlangen (DE); Knop, Sophia, 91052, Erlangen (DE); Use, Tim, 90469, Nürnberg (DE)

(56) Entgegenhaltungen:
- EP-A- 1 062 912
- US-B1- 6 207 952
- "2000 Optics and Optical Instruments Catalog" 2000, EDMUND INDUSTRIAL OPTICS CATALOG 2000 , BARRINGTON, N.J. , XP002486772 * Seite 106 *

## Beschreibung

Die Erfindung betrifft das Gebiet der Qualitätsüberprüfung bei einer Partikeltherapieanlage. Im Speziellen betrifft die Erfindung eine Vorrichtung und ein Verfahren zur Qualitätsüberprüfung einer Partikeltherapieanlage

Die Strahlentherapie wird insbesondere zur Bestrahlung von Tumorgewebe bei Menschen eingesetzt. Bei der Partikeltherapie, einer speziellen Form der Strahlentherapie, können zur Bestrahlung verschiedene Arten von Ionen eingesetzt werden, wie beispielsweise Protonen, Heliumionen, Pionen, Kohlenstoffionen oder auch andere Ionensorten. Die Bestrahlung mit Ionen kennzeichnet sich im Vergleich zu einer herkömmlichen Bestrahlung mit Photonen oder Elektronen dadurch aus, dass insbesondere die Eindringtiefe der Strahlung in das menschliche Gewebe genauer gesteuert werden kann. Hierdurch ist eine gezieltere Bestrahlung des Tumors möglich, bei der umliegendes, zu schonendes Gewebe besser vor Strahlung geschützt werden kann.

Die Ionen werden hierzu in einem Beschleunigersystem, das beispielsweise ein Synchrotron oder ein Zyklotron umfasst, auf hohe Energien beschleunigt und in einem Strahl auf den zu behandelnden Körper gerichtet. Der Strahl wird dabei durch ein Magnetsystem abgelenkt und gesteuert. Wesentliche und für die Sicherheit relevante Parameter des Strahls sind im Wesentlichen abhängig von den Einstellungen an der Anlage selbst, die als Resultat die Strahleigenschaften bestimmen. Hierzu gehören unter anderem der Strahldurchmesser, die Strahlposition und die Energie des Strahls.

Die Eigenschaften des bereitgestellten Partikelstrahls müssen bekannt sein. Um dies zu gewährleisten, werden in vorzugsweise regelmäßigen Abständen Qualitätssicherungsmaßnahmen (auch als QA-Maßnahmen bezeichnet, QA für engl. "quality assurance") durchgeführt, beispielsweise täglich oder wöchentlich.

Die Überprüfung der Strahleigenschaften wird unter anderem mit so genannten Phantomen durchgeführt. Derartige Phantome werden mit dem Partikelstrahl bestrahlt, wobei die Wechselwirkung des Partikelstrahls mit dem Phantom je nach eingesetztem Phantom auf unterschiedliche Art und Weise gemessen oder detektiert wird. Dies kann beispielsweise durch Filme geschehen, die durch die Bestrahlung mit dem Partikelstrahl belichtet werden und dadurch Rückschlüsse auf die Parameter des Partikelstrahls zulassen. Es können aber auch Detektoren eines anderen Typs, wie beispielsweise Ionisationskammern oder Fingerhutkammern, eingesetzt werden.

Die Überwachung verschiedener Strahlparameter erfordert den Einsatz vieler unterschiedlicher Phantome. Bisher werden diese einzeln und nacheinander entsprechend zum Strahl manuell positioniert. Hierdurch entstehen mehrere Nachteile. Für jede QA-Maßnahme muss das jeweils nötige Phantom in eine definierte Lage zum Strahl gebracht werden, so dass für unterschiedliche Phantome die Positionierung jeweils erneut durchgeführt werden muss und mehrmals ein manuelles Intervenieren erfordert. Der hier anfallende Zeit-, Personal- und Kostenaufwand ist erheblich.

Es ist die Verwendung von Platten mit einem Lochmuster als Tischplatte bekannt, auf welcher optische Geräte montiert werden können.

Die EP 1 062 912 A1 offenbart ein Phantom mit mehreren Unter-Phantomen, welches in einer Röntgenanlage eingesetzt wird.

Die US 6,207,852 offenbart ein Phantom, das über einen Befestigungsrahmen an eine Bestrahlungsvorrichtung einer Partikeltherapieanlage gehängt werden kann.

Es ist die Aufgabe der Erfindung, eine Vorrichtung zur Durchführung von QA-Maßnahmen bei einer Partikeltherapieanlage bereitzustellen, mit deren Hilfe QA-Maßnahmen auf einfach, kostengünstige und Zeit sparende Weise durchgeführt werden können. Weiterhin ist die Aufgabe der Erfindung ein Verfahren zur Qualitätsüberprüfung einer Partikeltherapieanlage, anzugeben, das auf einfache, kostengünstige und Zeit sparende Weise durchgeführt werden kann.

Demnach wird die Aufgabe der Erfindung durch eine Vorrichtung nach Anspruch 1 und durch ein Verfahren nach Anspruch 5 gelöst. Vorteilhafte Weiterbildungen finden sich in den Merkmalen der abhängigen Ansprüche.

Die erfindungsgemäße Vorrichtung umfasst eine Halterungsvorrichtung für Phantome, die zur Qualitätsüberprüfung einer Partikeltherapieanlage eingesetzt wird. Die Halterungsvorrichtung ist dabei derart ausgebildet, dass mehrere Phantome jeweils in einer definierten Position auf der Halterungsvorrichtung reproduzierbar platzierbar sind.

Mit dieser Halterungsvorrichtung ist es nun möglich, gleichzeitig mehrere verschiedene Phantome präzise und reproduzierbar auf der Halterungsvorrichtung zu positionieren. Die Positionierung der unterschiedlichen Phantome im Raum kann nun über die Positionierung der einen Halterungsvorrichtung erfolgen. Hierdurch vereinfacht sich die Qualitätsüberprüfung: Während ein manuelles und sukzessives Positionieren der Phantome im Raum aufwändig ist und ein kontinuierliches Eingreifen durch einen Anwender erfordert, können nun mehrere Phantome ein einziges Mal auf der Halterungsvorrichtung positioniert werden. Durch ein Positionieren der Halterungsvorrichtung im Raum können nun die verschiedenen Phantome auf einfachere Weise jeweils in die Position gebracht werden, in der dann die QA-Maßnahme durchgeführt wird. Die Anzahl und der Aufwand der manuellen Intervention werden dadurch reduziert.

Mit den unterschiedlichen Phantomen können je nach Ausgestaltung des Phantoms bestimmte Eigenschaften des Partikelstrahls überprüft werden, wie z.B. die Reichweite und damit die Energie des Partikelstrahls, die Ausdehnung bzw. die Strahlbreite des Partikelstrahls oder die Position des Partikelstrahls. Die Auswertung der überprüften Strahlparameter kann dabei je nach Ausbildung des Phantoms online, d.h. während der Durchführung der zugehörigen QA-Maßnahme, durchgeführt werden oder aber auch nach erfolgter Durchführung der zugehörigen QA-Maßnahme.

Dadurch, dass die Phantome reproduzierbar auf der Halterungsvorrichtung platzierbar sind, kann eine gleiche Anordnung von Phantomen auf einfache Weise wiederholt hergestellt werden, selbst wenn die Phantome ausgetauscht werden und/oder die Halterungsvorrichtung mit den Phantomen zerlegt wird.

Die Halterungsvorrichtung weist eine Fixiervorrichtung zum Anschluss an ein Positioniersystem auf. Das Positioniersystem ist in besonders vorteilhafter weise das Patientenpositioniersystem, das in Behandlungsräumen bei einer Strahlentherapieanlage bereits vorhanden ist. Durch die Fixiervorrichtung gelingt eine präzise und reproduzierbare Befestigung der Halterungsvorrichtung an das Positioniersystem. Die Positionierung der Halterungsvorrichtung mit den Phantomen im Raum wird darauf durch das Positioniersystem vorgenommen. Dies kann vorzugsweise automatisch geschehen, indem Steuerparameter für das Positioniersystem in einer Steuereinheit für das Positioniersystem hinterlegt werden.

Über die Fixiervorrichtung kann die Halterungsvorrichtung beispielsweise an einen Roboterarm angekoppelt werden, der sonst zur Positionierung eines Patiententisches verwendet wird. Die Ankopplung kann dabei direkt an den Roboterarm erfolgen, indem der Patiententisch entfernt wird, oder aber auch an den Patiententisch.

Die Steuerparameter sind auf die jeweilige Phantomanordnung auf der Halterungsvorrichtung abgestimmt, so dass jeweils eines der Phantome in eine für die entsprechende QA-Maßnahme passende Position im Raum positioniert werden kann. Dadurch, dass die Phantome reproduzierbar auf der Halterungsvorrichtung angeordnet werden können, müssen die Steuerparameter für die Halterungsvorrichtung lediglich dann neu bestimmt werden, wenn eine andere Anordnung der Phantome auf der Halterungsvorrichtung gewählt wird.

Die Halterungsvorrichtung ist als eine im Wesentlichen ebene Plattform ausgebildet. Eine ebene Plattform ist üblicherweise an die ebene Geometrie eines Patiententisches angepasst und kann dadurch z.B. auf besonders einfache Weise mit einer Fixiervorrichtung versehen werden, um die Plattform das Patientenpositioniersystem anzuschließen.

Die Plattform weist in einer Ausführungsform ein insbesondere regelmäßiges Lochmuster auf. Durch dieses Lochmuster können die Phantome beispielsweise durch Einrasten auf einfache Weise und reproduzierbar an eine definierte Position auf der Plattform gebracht werden.

In einer Weiterbildung weist die Halterungsvorrichtung zumindest eine Positionsmarkierung auf, mit deren Hilfe die korrekte Position der Halterungsvorrichtung im Raum überprüft werden kann. Da die definierte Lage der Phantome auf der Halterungsvorrichtung bekannt ist, kann hierüber auch auf indirekte Weise die Position der Phantome im Raum überprüft werden, wodurch sich die Qualität und die Sicherheit einer QA-Maßnahme erhöht.

Das erfindungsgemäße Verfahren zur Qualitätsüberprüfung einer Partikeltherapieanlage, weist folgende Schritte auf:
(a) Platzieren mehrerer Phantome auf einer Halterungsvorrichtung jeweils an einer vordefinierten Position,
(b) Positionieren der Halterungsvorrichtung in der Strahlentherapieanlage, so dass sich eines der Phantome in einer für die Durchführung einer Qualitätsüberprüfung vorgesehenen Position befindet,
(c) Durchführen der Qualitätsüberprüfung mit dem Phantom,
(d) Wiederholen der Schritte (b) und (c), bis mit jedem der Phantome eine Qualitätsüberprüfung durchgeführt wurde.

Durch die Verwendung einer Halterungsvorrichtung für mehrere Phantome vereinfacht sich das Verfahren zur Qualitätsüberprüfung insgesamt. Die Positionierung der Phantome erfolgt über eine sukzessive Positionierung der einen Halterungsvorrichtung, so dass bei der Durchführung der Qualitätsüberprüfung das wiederholte manuelle Austauschen der einzelnen Phantome entfällt. Durch die Automatisierung der sukzessiven Positionierung verschiedener Phantome ergibt sich eine deutliche Zeitersparnis.

In einer vorteilhaften Weiterbildung wird die Halterungsvorrichtung von einem Positioniersystem, insbesondere von einem Patientenpositioniersystem, positioniert. Hierüber kann die Positionierung der Halterungsvorrichtung im Raum jeweils besonders einfach, automatisch und genau durchgeführt werden. Die Steuerparameter für das Positioniersystem können dabei insbesondere in einer Steuereinheit hinterlegt und jeweils der vordefinierten Position der Phantome auf der Halterungsvorrichtung angepasst sein. Hiermit lässt sich die Durchführung mehrerer QA-Maßnahmen weit gehend automatisieren, es bedarf lediglich einer einmaligen Fixierung der Halterungsvorrichtung an das Positioniersystem.

In einer vorteilhaften Ausführungsform wird nach jedem Positionieren der Halterungsvorrichtung die Position der Halterungsvorrichtung oder des jeweiligen Phantoms relativ zu einem raumfesten Koordinatensystem insbesondere mit Hilfe einer Laservorrichtung überprüft. Dies kann beispielsweise ein System aus Laserfächern sein, die einen speziellen Koordinatenpunkt im Raum anzeigen, beispielsweise das Isozentrum. Die Laserfächer können aber auch Koordinatenpunkte im Raum anzeigen, die jeweils die Sollposition der Positionsmarkierung der Halterungsvorrichtung anzeigen. Die Überprüfung der korrekten Position kann beispielsweise visuell oder automatisch mit geeigneten Detektoren erfolgen. Es besteht aber auch die Möglichkeit, die Koordinaten des Positioniersystems an einer Bedienkonsole zu verfolgen und zu überprüfen.

Ausführungsformen der Erfindung und Weiterbildungen gemäß den Merkmalen der abhängigen Ansprüche werden nun anhand der folgenden Zeichnung näher erläutert, ohne jedoch darauf beschränkt zu sein. Es zeigen:
- Figur 1: einen schematischen Überblick über den Aufbau einer Partikeltherapieanlage,
- Figur 2: eine perspektivische Ansicht einer Halterungsvorrichtung für Phantome,
- Figur 3: eine Draufsicht auf eine Halterungsvorrichtung, auf der mehrere Phantome angeordnet sind und
- Figur 4: einen schematischen Überblick über die Schritte eines Verfahrens für die Qualitätsüberprüfung.

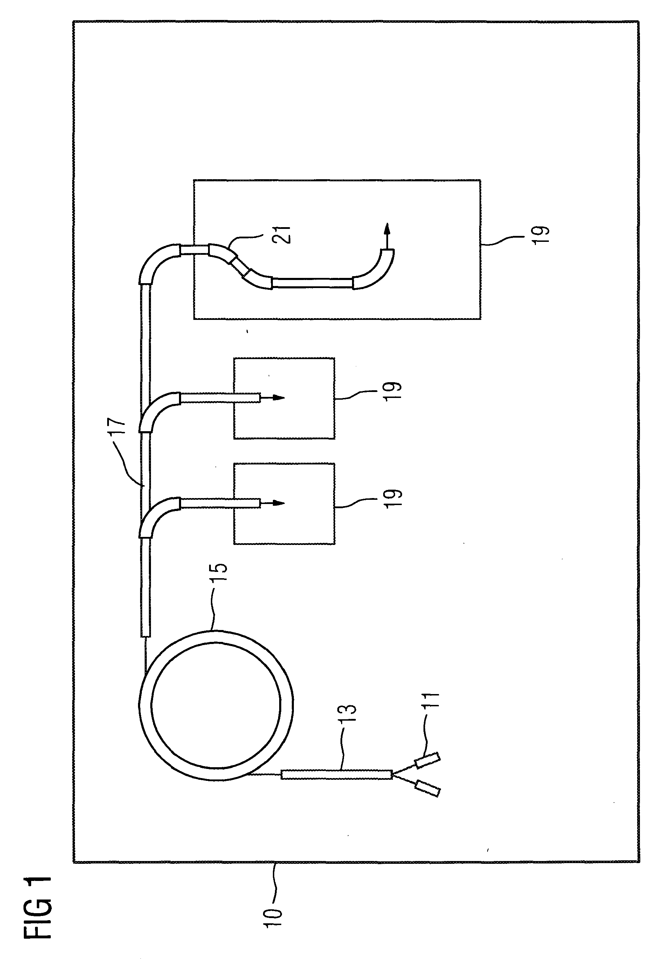

Figur 1 zeigt einen schematischen Überblick über den Aufbau einer Partikeltherapieanlage 10. In einer Partikeltherapieanlage 10 erfolgt die Bestrahlung insbesondere von tumorerkranktem Gewebe mit einem Partikelstrahl. Als Partikel werden vornehmlich Ionen wie beispielsweise Protonen, Pionen, Heliumionen, Kohlenstoffionen oder andere Ionensorten eingesetzt.

Üblicherweise werden derartige Partikel in einer Partikelquelle 11 erzeugt und in einem Vorbeschleuniger 13, z.B. einem linearen Beschleuniger (LINAC für engl.: "LINear ACcelerator"), beschleunigt. Anschließend werden die Partikel in einen Beschleuniger 15, beispielsweise in ein Synchrotron oder Zyklotron, eingespeist, in dem sie auf hohe Energien, wie sie zur Bestrahlung nötig sind, beschleunigt werden. Nachdem die Partikel den Beschleuniger 15 verlassen, führt ein Hochenergiestrahl-Transportsystem 17 den Partikelstrahl zu den gewünschten Bestrahlungsräumen 19. In einem Bestrahlungsraum 19 werden die beschleunigten Partikel auf einen Körper gerichtet. Je nach Ausgestaltung erfolgt dies von einer festen Richtung (in so genannten "fixed beam"-Räumen) aus oder aber über eine bewegliche rotierbare Gantry 21 von verschiedenen Richtungen aus. Dieser Grundaufbau einer Partikeltherapieanlage 10 ist typisch für viele Partikeltherapieanlagen, kann aber auch hiervon abweichen.

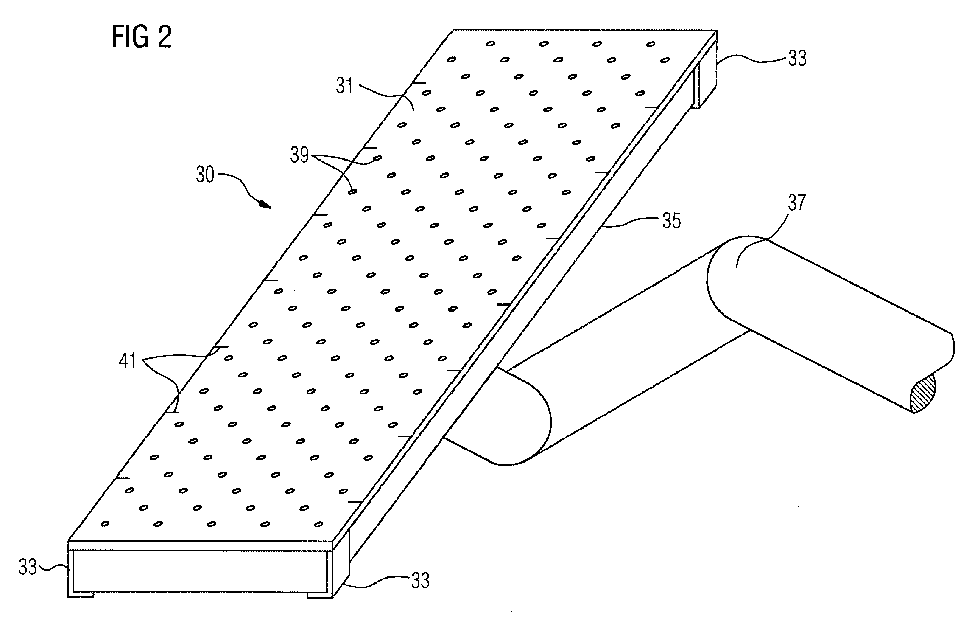

Figur 2 zeigt eine perspektivische Ansicht einer Halterungsvorrichtung 30 für Phantome, auf der verschiedene Phantome an einer vordefinierten Position angeordnet werden können. Die Halterungsvorrichtung 30 ist als eine im Wesentlichen ebene Plattform 31 ausgebildet. In dem hier gezeigten Beispiel weist die Halterungsvorrichtung 30 weist an ihren Ecken jeweils Fixiervorrichtungen 33 auf, mit denen die Halterungsvorrichtung 30 an einen Patiententisch 35 lösbar angekoppelt werden kann. Der Patiententisch 35 selbst ist im Bestrahlungsraum über ein Positioniersystem 37 positionierbar. Das Positioniersystem 37 kann z.B. als Roboterarm ausgebildet sein und an der Unterseite an den Patiententisch 35 andocken.

Über das Positioniersystem 37, in diesem Falle das Patientenpositioniersystem, kann der Patiententisch 35 im Behandlungsraum an einen gewünschten Ort im Behandlungsraum positioniert werden.

Die hier gezeigte Ausbildung der Fixiervorrichtung 33 als Umklammerung für den Patiententisch 35 ist lediglich eine von vielen Möglichkeiten.

Die Halterungsvorrichtung kann alternativ auch so ausgebildet sein, dass sie direkt an das Positioniersystem für den Patiententisch 35 andocken kann. In diesem Fall wird der Patiententisch 35 zunächst entfernt; die Halterungsvorrichtung 30 wird daraufhin über eine beispielsweise an der Unterseite der Halterungsvorrichtung 30 angebrachte Fixiervorrichtung an das Positioniersystem für die Patientenliege angekoppelt.

Über das Positioniersystem 37 kann nun auch die Halterungsvorrichtung 30 an eine vordefinierte Position im Raum gefahren werden. Über die Fixiervorrichtung 33 lässt sich die Halterungsvorrichtung 30 stets in gleicher Relativposition zu dem Patiententisch 35 ausrichten.

Das Andocken des Positioniersystems 37, das beispielsweise als Roboterarm ausgebildet sein kann, an die Halterungsvorrichtung 30 kann insbesondere auch automatisch ausgeführt werden. Beispielsweise kann die Halterungsvorrichtung 30 auf einem Shuttle zwischengelagert sein, so dass ein Anwender die Halterungsvorrichtung auf einfache Weise in den Bestrahlungsraum bringen kann. Das Positioniersystem 37 kann daraufhin automatisch an die Halterungsvorrichtung 30 andocken, die Halterungsvorrichtung 30 von dem Shuttle abnehmen und an die gewünschte Position im Raum bringen.

Die ebene Plattform 31 der Halterungsvorrichtung 30 weist eine Vielzahl von lochförmigen Vertiefungen 39 auf, die in einem regelmäßigen Muster über die ebene Plattform 31 verteilt sind. Die Vertiefungen 39 werden dazu verwendet, Phantome auf der ebenen Plattform in einer vordefinierten Position anzuordnen. Hierzu können die Phantome an der Unterseite beispielsweise Vorsprünge aufweisen, die an die Vertiefungen angepasst sind und mit denen die Phantome auf einfache Weise reproduzierbar an die gleiche Position auf der ebenen Plattform 31 angeordnet werden können.

Weiterhin kann die ebene Plattform 31 Positionsmarkierungen 41 aufweisen, mit denen eine korrekte Positionierung der Halterungsvorrichtung 30 im Raum überprüft werden kann. Dies kann beispielsweise durch ein System von Laserfächern erreicht werden, die im Behandlungsraum angeordnet sind, und mit denen ein bestimmter Punkt im Behandlungsraum markiert werden kann. Die korrekte Positionierung der Halterungsvorrichtung 30 im Raum kann daraufhin durch einen visuellen oder automatischen Abgleich der Positionsmarkierungen 41 mit den Laserfächern überprüft werden.

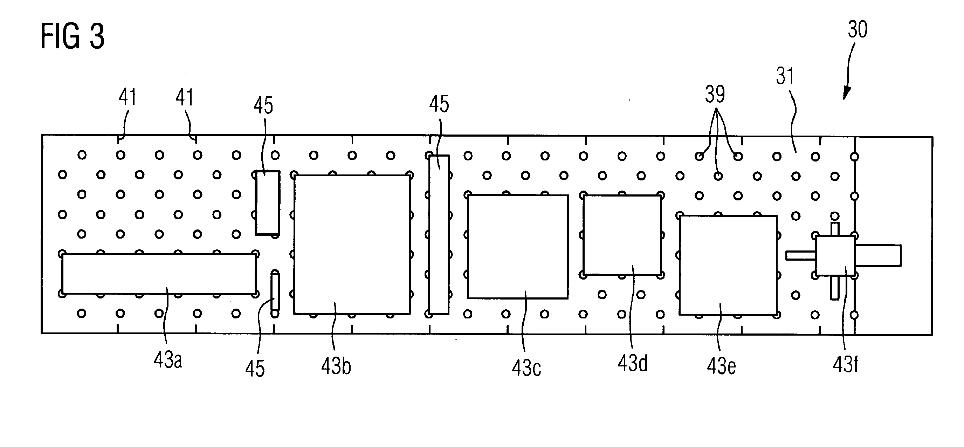

Figur 3 zeigt eine Draufsicht auf die ebene Plattform 31, auf der verschiedenen Phantome 43a ... 43f angeordnet sind, die jeweils zur Durchführung einer speziellen QA-Maßnahme eingesetzt werden. Auf der ebenen Plattform 31 können neben den Phantomen 43a ... 43f auch weitere Komponenten 45, die zum Betrieb der Phantome notwendig sind, angeordnet werden, wie z.B. Netzteile, Wandler, Steckerleisten etc.

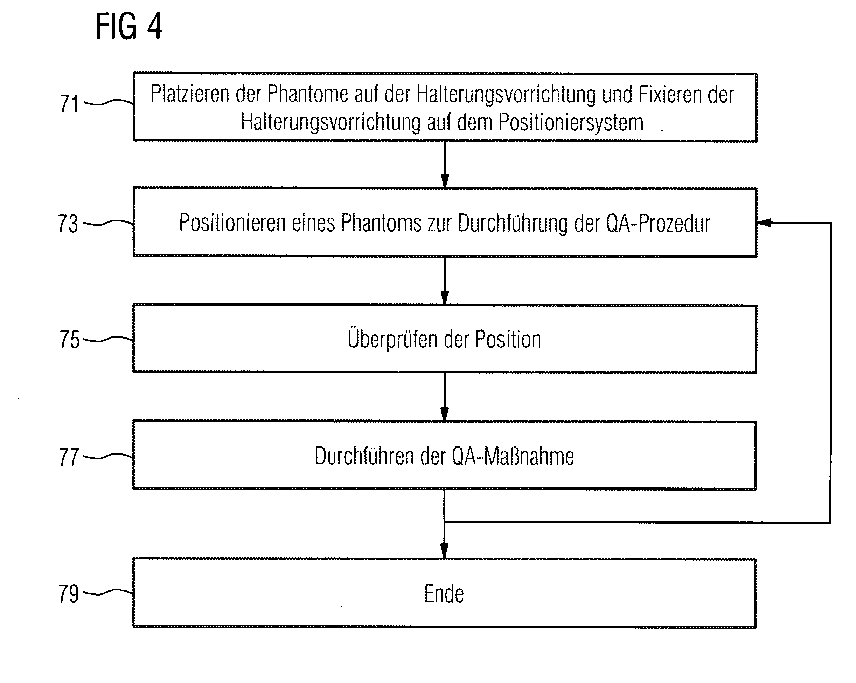

Figur 4 zeigt einen schematischen Überblick über die Schritte eines Verfahrens zur Qualitätsüberprüfung.

In einem ersten Schritt 71 werden die Phantome auf der Halterungsvorrichtung positioniert. Die Positionierung der Phantome auf der Halterungsvorrichtung kann einmalig vorgenommen werden und solange belassen werden, bis beispielsweise ein einzelnes Phantom ausgetauscht werden muss. Für die Durchführung von den QA-Maßnahmen mit den Phantomen ist damit lediglich eine einmalige manuelle Positionierung der Halterungsvorrichtung mit den Phantomen (siehe Figur 3) auf einem Positioniersystem notwendig. Das restliche Verfahren der QA-Maßnahmen kann dann wie folgt beschrieben automatisch durchgeführt werden.

In einem zweiten Schritt 73 wird die Halterungsvorrichtung mithilfe des Positioniersystems derart im Raum positioniert, dass sich eines der Phantome an der für die Durchführung der QA-Maßnahme notwendigen Stelle im Behandlungsraum befindet. Hierzu sind die notwendigen Steuerparameter in einer Steuereinheit für das Positioniersystem hinterlegt.

In einem dritten Schritt 75 erfolgt optional ein weiteres Überprüfen der Position, beispielsweise mit den in Figur 2 beschriebenen Positionsmarkierungen oder mit einem im Behandlungsraum angeordneten Lasersystem zur Projektion von Laserfächern, die bestimmte Koordinaten im Raum anzeigen.

In einem vierten Schritt 77 erfolgt nun die Durchführung der QA-Maßnahme. Der zweite bis vierte Schritt 73 ... 75 werden solange wiederholt, bis alle QA-Maßnahmen mit allen Phantomen durchgeführt worden sind (Ende 79).

## Patentansprüche

1. Vorrichtung für die Qualitätsüberprüfung einer Partikel therapieanlage, umfassend:
- mehrere verschiedene Phantome, die zur die Qualitätsüberprüfung einer Partikeltherapieanlage eingesetzt werden und mit denen jeweils unterschiedliche Eigenschaften eines Partikelstrahls überprüfbar sind,
- eine Halterungsvorrichtung für Phantome, wobei die Halterungsvorrichtung als eine im Wesentlichen ebene Plattform derart ausgebildet ist, dass die mehreren verschiedenen Phantome jeweils in einer definierten Position auf der Halterungsvorrichtung reproduzierbar gleichzeitig platzierbar sind, und
- ein Positioniersystem für die Halterungsvorrichtung,
wobei die Halterungsvorrichtung eine Fixiervorrichtung zum Anschluss an das Positioniersystem aufweist.

2. Vorrichtung nach Anspruch 1, wobei die Plattform ein insbesondere regelmäßiges Lochmuster zum Einrasten der Phantome an den definierten Positionen aufweist.

3. Vorrichtung nach einem der Ansprüche 1 bis 2, wobei die Halterungsvorrichtung zumindest eine Positionsmarkierung aufweist.

4. Partikeltherapieanlage, umfassend
- ein Patientenpositioniersystem und
- eine Vorrichtung nach einem der Ansprüche 1 bis 3,
wobei das Patientenpositioniersystem das Positioniersystem für die Halterungsvorrichtung ist.

5. Verfahren zur Qualitätsüberprüfung einer Partikel therapieanlage, aufweisend folgende Schritte:
(a) Platzieren mehrerer Phantome auf einer Halterungsvorrichtung jeweils an einer vordefinierten Position, wobei mit den verschiedenen Phantomen jeweils unterschiedliche Eigenschaften eines Partikelstrahls überprüfbar sind,
(b) Positionieren der Halterungsvorrichtung in der Partikel therapieanlage, so dass sich eines der Phantome in einer für die Durchführung einer Qualitätsüberprüfung vorgesehenen Position befindet,
(c) Durchführen der Qualitätsüberprüfung mit dem Phantom,
(d) Wiederholen der Schritte (b) und (c), bis mit jedem der Phantome eine Qualitätsüberprüfung durchgeführt wurde.

6. Verfahren nach Anspruch 5, wobei die Halterungsvorrichtung von einem Positioniersystem, insbesondere von einem Patientenpositioniersystem, positioniert wird.

7. Verfahren nach Anspruch 6, wobei Steuerparameter zum Positionieren der Halterungsvorrichtung mit dem Positioniersystem in einer Steuereinheit für das Positioniersystem hinterlegt sind.

8. Verfahren nach einem der Ansprüche 5 bis 7, wobei nach jedem Positionieren der Halterungsvorrichtung die Position der Halterungsvorrichtung relativ zu einem raumfesten Koordinatensystem insbesondere mit Hilfe zumindest einer Positionsmarkierung, die die Halterungsvorrichtung aufweist, überprüft wird.

## Claims

1. Device for quality testing a particle therapy assembly, comprising:
- a number of different phantoms, which are used for quality testing a particle therapy assembly and with which different properties of a particle beam can be tested in each instance,
- a holding device for phantoms, with the holding device being embodied as an essentially flat platform such that the a number of different phantoms can at the same time be placed in a reproducible fashion in a defined position on the holding device, and
- a positioning system for the holding device, with the holding device having a fixing device for connection to the positioning system.

2. Device according to claim 1, with the platform especially having an regular hole pattern for latching the phantoms into the defined positions.

3. Device according to one of claims 1 to 2, with the holding device having at least one position marker.

4. Particle therapy assembly, comprising:
- a patient positioning system and
- a device according to one of claims 1 to 3, with the patient positioning system being the positioning system for the holding device.

5. Method for quality testing a particle therapy assembly, comprising the following steps:
a) placing a number of phantoms on a holding device at a predefined position in each instance, with different properties of a particle beam being testable using the different phantoms in each instance,
b) positioning the holding device in the particle therapy assembly so that one of the phantoms is in a position provided for the implementation of a quality test,
c) implementing the quality test with the phantom,
d) repeating the steps (b) and (c) until a quality test has been carried out with each of the phantoms.

6. Method according to claim 5, with the holding device being positioned by a positioning system, in particular by a patient positioning system.

7. Method according to claim 6, with control parameters for positioning the holding device with the positioning system being stored in a control unit for the positioning system.

8. Method according to one of claims 5 to 7, with, after each positioning of the holding device, the position of the holding device being tested relative to a spatially fixed coordinate system, in particular with the aid of at least one position marker appearing on the holding device.

## Revendications

1. Dispositif de vérification de la qualité d'une installation de thérapie à particules, comprenant .
- plusieurs fantômes différents qui sont utilisés pour vérifier la qualité d'une installation de thérapie à particules et à l'aide desquels des caractéristiques à chaque fois différentes d'un faisceau de particules peuvent être vérifiées,
- un dispositif de maintien pour les fantômes, le dispositif de maintien étant exécuté en tant que plate-forme essentiellement plane de manière à ce que les plusieurs fantômes différents puissent être placés simultanément à chaque fois dans une position définie sur le dispositif de maintien d'une manière reproductible et
- un système de positionnement pour le dispositif de maintien, le dispositif de maintien comprenant un dispositif de fixation pour la connexion au système de positionnement.

2. Dispositif selon la revendication 1, la plate-forme présentant un motif à trous, notamment régulier, permettant d'engager les fantômes au niveau des positions définies.

3. Dispositif selon l'une des revendications 1 à 2, le dispositif de maintien possédant au moins une marque de position.

4. Installation de thérapie à particules, comprenant .
- un système de positionnement de patient et
- un dispositif selon l'une des revendications 1 à 3, le système de positionnement de patient étant le système de positionnement pour le dispositif de maintien.

5. Procédé de vérification de la qualité d'une installation de thérapie à particules, comprenant les étapes suivantes :
(a) mise en place de plusieurs fantômes sur un dispositif de maintien à chaque fois au niveau d'une position prédéfinie, des caractéristiques à chaque fois différentes d'un faisceau de particules pouvant être vérifiées à l'aide des différents fantômes,
(b) positionnement du dispositif de maintien dans l'installation de thérapie à particules, de manière à ce que l'un des fantômes se trouve dans une position prévue pour procéder à une vérification de la qualité,
(c) exécution de la vérification de la qualité avec le fantôme,
(d) répétition des étapes (b) et (c) jusqu'à ce qu'une vérification de la qualité ait été exécutée avec chacun des fantômes.

6. Procédé selon la revendication 5, le dispositif de maintien étant positionné par un système de positionnement, notamment par un système de positionnement de patient.

7. Procédé selon la revendication 6, des paramètres de commande pour le positionnement du dispositif de maintien par le système de positionnement étant mémorisés dans une unité de commande pour le système de positionnement.

8. Procédé selon l'une des revendications 5 à 7, la position du dispositif de maintien par rapport à un système de coordonnées fixe dans l'espace étant vérifiée après chaque positionnement du dispositif de maintien, à l'aide notamment d'au moins une marque de position que possède le dispositif de maintien.
